(19) Europäisches Patentamt
European Patent Office
Office européen des brevets

(11) Veröffentlichungsnummer: **0 129 158**
**A2**

(12) # EUROPÄISCHE PATENTANMELDUNG

(21) Anmeldenummer: 84106514.7

(22) Anmeldetag: 07.06.84

(51) Int. Cl.$^3$: **C 07 C 91/26**

(30) Priorität: 15.06.83 DE 3321608

(43) Veröffentlichungstag der Anmeldung:
27.12.84 Patentblatt 84/52

(84) Benannte Vertragsstaaten:
AT BE CH DE FR GB IT LI LU NL SE

(71) Anmelder: Henkel Kommanditgesellschaft auf Aktien
Postfach 1100 Henkelstrasse 67
D-4000 Düsseldorf-Holthausen(DE)

(72) Erfinder: Rutzen, Horst, Dr.
Falkenweg 12
D-4018 Langenfeld(DE)

(72) Erfinder: Boutty, Bernd
Ilbertzweg 1
D-4005 Meerbusch 1(DE)

(54) **Verfahren zur Herstellung von quartären Ammoniumverbindungen.**

(57) Quartäre Ammoniumverbindungen mit wenigstens einem langkettigen Hydroxyalkylrest werden hergestellt, indem man das Salz aus einem tertiären Amin und einer schwachen Säure mit einer den langkettigen Hydroxyalkylrest einführenden Epoxidverbindung in stöchiometrischem Verhältnis in Wasser bei erhöhter Temperatur unter Normaldruck umsetzt. Die wäßrige Phase des zweiphasigen Reaktionsgemisches weist vor Beginn der Umsetzung einen pH-Wert von wenigstens 7 auf.

EP 0 129 158 A2

4000 Düsseldorf, den 13.06.1983
Henkelstraße 67

0129158

HENKEL KGaA
ZR-FE/Patente
Dr. Ms/Ne

P a t e n t a n m e l d u n g

D 6713 EP

"Verfahren zur Herstellung von quartären Ammoniumverbindungen"

Zur Herstellung von quartären Ammoniumverbindungen geht man meist von einem tertiären Amin aus, das zur Quartärstufe alkyliert wird. Als Alkylierungsmittel läßt man dabei üblicherweise Ester starker Mineralsäuren, insbesondere Alkylhalogenide, Schwefel- oder Sulfonsäureester auf tertiäre Amine einwirken; gelegentlich werden auch andere Ester benutzt. Eine weitere bekannte Möglichkeit zur Alkylierung tertiärer Amine stellt die Reaktion von niederen Alkylenoxiden mit tertiären Aminen in Gegenwart von Wasser dar. Darüber hinaus gibt es noch eine Reihe anderer Möglichkeiten zur Herstellung von quartären Ammoniumverbindungen aus den leicht zugänglichen tertiären Aminen (vgl. J. Goerdeler in Houben-Weyl, Methoden der organischen Chemie, 4. Auflage, Band 11/2, Seite 592 ff.). Quartäre Ammoniumverbindungen mit einem oder mehreren langen aliphatischen Resten weisen antimikrobielle beziehungsweise textilweichmachende und antistatischmachende Eigenschaften auf und finden breite Anwendung. Man erhält derartige Verbindungen, indem man entweder tertiäre Amine mit langen aliphatischen Resten alkyliert oder die Alkylierung mit Alkylierungsmitteln vornimmt, die ihrerseits lange aliphatische Reste enthalten, wobei

...

natürlich auch sowohl im tertiären Amin als auch im Alkylierungsmittel lange aliphatische oder aromatische Reste vorhanden sein können. Ein Nachteil der bekannten Verfahren zur Herstellung der quartären Ammoniumverbindungen ist, daß häufig unter Druck gearbeitet werden muß, gelegentlich Lösungsmittel erforderlich sind und die Ausbeute meist unbefriedigend ist.

In der DE-OS 31 36 564 ist ein Verfahren zur Herstellung von quartären Ammoniumverbindungen beschrieben, bei dem man tertiäre Amine, die teilweise in Salzform vorliegen, in wäßriger Phase mit längerkettigen Epoxiden, die wenigstens eine endständige Epoxygruppe aufweisen, bei erhöhter Temperatur unter Normaldruck umsetzt und anschließend in einem weiteren Verfahrensschritt die zur vollständigen Salzbildung benötigte Säuremenge dem Umsetzungsprodukt hinzufügt.

Es wurde nun ein Verfahren zur Herstellung von wenigstens einen langkettigen Hydroxyalkylrest enthaltenden quartären Ammoniumverbindungen durch Umsetzen von Salzen tertiärer Amine in wäßriger Phase bei erhöhter Temperatur mit Epoxidverbindungen, die wenigstens eine endständige Epoxygruppe aufweisen, gefunden, bei welchem man ohne den Verfahrensschritt der Vervollständigung der Salzbildung auskommt. Dieses Verfahren ist dadurch gekennzeichnet, daß man das Salz eines tertiären Amins und einer anorganischen oder organischen Säure in Wasser mit einer den Hydroxyalkylrest einführenden Epoxidverbindung, die wenigstens eine endständige Epoxygruppe und wenigstens 6 Kohlenstoffatome aufweist, in stöchiometrischem Verhältnis bei Normaldruck und einer Temperatur zwischen 40 und 100 °C umsetzt, wobei die wäßrige Phase des zweiphasigen Reaktionsgemisches vor Beginn der Umsetzung einen pH-Wert von wenigstens 7 aufweist.

...

Als Säure sind im Rahmen dieser Erfindung solche Säuren oder saure Salze zu verstehen, die in einem Reaktionsgemisch, enthaltend pro Liter 1 Mol 1,2-Epoxydodecan, 1 Mol Amin, eine dem Amin äquivalente Säuremenge oder Menge eines sauren Salzes, Rest Wasser, in der wäßrigen Phase nach gründlichem Vermischen bei 20 °C zu einem pH-Wert von wenigstens 7 führen. In Fällen, in denen das Reaktionsgemisch ein Amin mit mehr als einem tertiären Stickstoffatom enthält, wird ein entsprechender Bruchteil des Amins bzw. des Aminsalzes eingesetzt, beispielsweise enthält das Reaktionsgemisch 1/2 Mol eines Disalzes eines ditertiären Amins. Typische bevorzugte Säuren, die unter die obige Definition fallen, sind z. B. Borsäure, Kohlensäure, Phosphorsäure, Phenol, Caprinsäure und saure Salze der Kohlensäure, Phosphorsäure und der schwefligen Säure; der pK-Wert der genannten Säuren liegt zwischen etwa 4 und etwa 13.

Geeignete Amine, die vorzugsweise für das erfindungsgemäße Verfahren Verwendung finden, sind Amine mit bis zu 3 tertiären Stickstoffatomen im Molekül, insbesondere 1 oder 2 tertiären Stickstoffatomen. Die tertiären Amine können an jedem Stickstoffatom eine aliphatische Alkyl- oder Alkenyl-Gruppe mit etwa 10 bis 20 Kohlenstoffatomen und im übrigen Alkyl-, Hydroxyalkyl-, Alkenyl- oder Alkylen-Gruppen mit höchstens 4, insbesondere höchstens 3 Kohlenstoffatomen, enthalten, wobei Amine mit Stickstoff-Substituenten mit ausschließlich kurzen aliphatischen Gruppen mit höchstens 4 Kohlenstoffatomen bevorzugt sind. Beispiele für besonders bevorzugte tertiäre Amine sind Dimethylethanolamin und Tetramethylpropylendiamin. Ein Beispiel für ein tertiäres Amin mit einem aliphatischen $C_{10}$- bis $C_{20}$-Rest ist Lauryldimethylamin. Auch tertiäre Amine mit einer aromatischen Gruppe sind geeignet.

...

Die für die Umsetzung nach dem erfindungsgemäßen Verfahren geeigneten 1,2-Epoxyalkane werden in bekannter Weise aus den entsprechenden 1,2-Monoolefinen bzw. Olefin-Gemischen erhalten, die beispielsweise durch Polymerisation von Ethylen mit organischen Aluminiumverbindungen als Katalysatoren oder durch thermisches Cracken von Paraffinkohlenwasserstoffen erhalten werden.

Die bevorzugten Epoxidverbindungen entsprechen entweder der allgemeinen Formel I

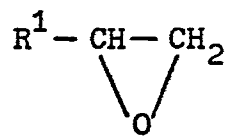 (I)

in der $R^1$ eine lineare oder verzweigte aliphatische Kohlenwasserstoffgruppe mit 4 bis 24 Kohlenstoffatomen oder eine Gruppe der allgemeinen Formel II

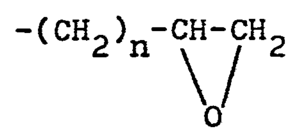 (II)

mit n = 4 bis 16 bedeutet, oder sie entsprechen einem Glycidether der allgemeinen Formel III

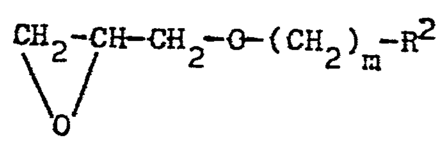 (III)

in der m einen Wert von 1 bis 10 und $R^2$ Wasserstoff oder eine aliphatische Kohlenwasserstoffgruppe mit 1 bis 24 Kohlenstoffatomen oder eine Gruppe der allgemeinen Formel IV

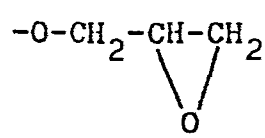 (IV)

bedeutet, mit der Maßgabe, daß der Glycidether der ...

Formel III wenigstens 6 Kohlenstoffatome enthält.

Beispiele für bevorzugte Epoxyalkane sind die Verbindungen 1,2-Epoxyhexan, 1,2-Epoxyoctan, 1,2-Epoxydecan, 1,2-Epoxydodecan, 1,2-Epoxytetradecan, 1,2-Epoxyhexadecan, 1,2-Epoxyoctadecan. Ebenfalls geeignet sind Epoxidgemische, wie zum Beispiel $C_{12/14}$-1,2-Epoxyalkan mit zirka 70 Gewichtsprozent $C_{12}$- und zirka 30 Gewichtsprozent $C_{14}$-Epoxyalkan oder $C_{16/18}$-1,2-Epoxyalkan mit zirka 40 Gewichtsprozent $C_{16}$- und zirka 60 Gewichtsprozent $C_{18}$-Epoxyalkan. Weiterhin bevorzugt sind Diepoxyalkane mit 8 bis 20 Kohlenstoffatomen und zwei endständigen Epoxygruppen, wie beispielsweise 1,2-7,8-Diepoxyalkane 1,2-9,10-Diepoxyalkane und ähnliche Verbindungen. Auch Mono- oder Diglycidether wie Hexadecylmonoglycidether und 1,4-Butandioldiglycidether sind bevorzugte Epoxidverbindungen mit endständigen Epoxidgruppen.

Entsprechend der Definition für die Epoxidverbindungen, durch welche die langkettigen Hydroxyalkylreste in die quartären Ammoniumverbindungen eingeführt werden, bedeutet "langkettige Hydroxyalkylreste" im Rahmen der vorliegenden Erfindung, daß die Hydroxyalkylreste wenigstens 6 Kohlenstoffatome aufweisen.

Das Aminsalz wird zur Umsetzung mit der Epoxidverbindung vorzugsweise in Wasser gelöst eingesetzt. Man kann dabei das Aminsalz in einer ausreichenden Menge Wasser auflösen

...

oder die Bildung des Salzes vornehmen, indem man zu dem in Wasser gelösten Amin die Säure hinzufügt und dann die so gebildete Aminsalzlösung mit der Epoxidverbindung umsetzt. Als eine günstige Reaktionstemperatur hat sich 40 bis 100 °C erwiesen, wobei eine Temperatur zwischen 80 und 95 °C sich als besonders günstig herausgestellt hat.

Das neue Verfahren zeichnet sich gegenüber den Verfahren des Standes der Technik dadurch aus, daß es in nur einem Verfahrensschritt die gewünschten quartären Ammoniumverbindungen in hoher Reinheit mit großen Ausbeuten liefert.

Erfindungsgemäß hergestellte Reaktionsprodukte mit einem langkettigen Hydroxyalkylrest und einem $C_{10}$- bis $C_{20}$-Alkylrest oder einem aromatischen Rest oder zwei Alkyl- oder Alkenyl-Resten mit höchstens etwa 10 Kohlenstoffatomen und im übrigen kurzkettigen Alkyl-Resten weisen antimikrobielle Eigenschaften auf. Mit zunehmender Anzahl und Kettenlänge von langkettigen Resten weisen die Reaktionsprodukte zunehmend ausgeprägte textilweichmachende und antistatischmachende Eigenschaften auf. Je nach chemischer Konstitution der Verbindungen sind gleitende Übergänge zu beobachten.

Besonders wertvolle Verbindungen mit einem $C_{10}$- bis $C_{20}$-Alkyl- oder Alkenyl-Rest und einem $C_{10}$- bis $C_{20}$-Hydroxyalkyl-Rest und im übrigen kurzen Resten erhält man, indem man ein tertiäres Amin, das sich von $C_{10}$- bis $C_{20}$-Fettsäuren ableitet und im übrigen zwei $C_1$- bis $C_4$-Alkylgruppen aufweist, mit einem $C_{10}$- bis $C_{20}$-Epoxyalkan umsetzt. Ein Verfahren zur Herstellung derartiger

...

Verbindungen ist daher ein bevorzugter Gegenstand der vorliegenden Erfindung. Die Verwendung der Verfahrensprodukte als Textilweichmacher oder -antistatika bzw. als Antimikrobika ist ein weiterer Gegenstand der Erfindung. Eine Verwendung erfolgt beispielsweise in flüssigen Mitteln zur Wäschenachbehandlung, die neben dem erfindungsgemäß hergestellten Wirkstoff zusätzlich wenigstens eine weitere Verbindung aus der Gruppe Trägermittel, Lösungsmittel, Verdünnungsmittel, Emulgator, Farbstoff, Duftstoff, Konservierungsmittel, Viskositätsstellmittel, Trübungsmittel und eventuell weitere übliche Zusatzstoffe enthalten.

Mittel zur Wäschenachbehandlung haben beispielsweise folgende Zusammensetzung:

| | |
|---|---|
| 2 bis 80 Gew.-% | erfindungsgemäß hergestellte Verbindungen mit je 1 langen $C_{10}$- bis $C_{20}$-Hydroxyalkyl- und $C_{10}$- bis $C_{20}$-Alkyl- oder Alkenyl-Rest, |
| 20 bis 98 Gew.-% | Träger, Lösungsmittel, Verdünnungsmittel, |
| 0 bis 20 Gew.-% | Emulgator, |
| 0 bis 3 Gew.-% | Konservierungsmittel, |
| 0 bis 5 Gew.-% | Duftstoff, |
| 0 bis 1 Gew.-% | Farbstoff, |
| Rest | Viskositätsstellmittel, Trübungsmittel, gegebenenfalls saure Verbindungen und sonstige übliche Zusatzstoffe. |

...

Auch wenn man die Verfahrensprodukte zu entsprechend zusammengesetzten Waschmitteln, die wenigstens eine waschaktive Verbindung enthalten, hinzufügt, können sie eine weichmachende Wirkung entfalten. Derartige Waschmittel basieren vorzugsweise auf Rezepturen mit nichtionischen Tensiden. Trägt man die Verfahrensprodukte gegebenenfalls zusammen mit üblichen Hilfs- und Zusatzstoffen auf textile Flächengebilde als Träger auf, können diese auch als Tumblerhilfsmittel Verwendung finden.

...

## B E I S P I E L E

Die folgenden Beispiele beschreiben das erfindungsgemäße Verfahren, bei dem mit unterschiedlichen Ausgangsstoffen unterschiedliche Verfahrensprodukte hergestellt wurden. Die Verfahrensweise war wie folgt: Die 1/2 Mol Epoxid äquivalente Menge Amin wurde mit Wasser verdünnt. Zu der wäßrigen Aminlösung wurde die dem Amin äquivalente Menge Säure gegeben und alles gründlich vermischt. Anschließend fügte man die dem Amin äquivalente Menge Epoxyverbindung hinzu, temperierte die zweiphasige Mischung auf 20 °C und füllte mit Wasser von 20 °C auf 500 ml Reaktionsansatz auf. Man ließ 6 Stunden bei 95 °C unter Rühren reagieren und verfolgte die Epoxidzahl als Maß für die Umsetzung im Verlauf der Reaktion.

### Beispiel 1

44,6 g (0,5 Mol) Dimethylethanolamin wurden mit insgesamt 324,9 g Wasser verdünnt und mit 31,0 g (0,5 Mol) Borsäure vermischt. Man erhielt eine klare, farblose Lösung. Nach Erhitzen der Lösung auf 95 °C setzte man 94,0 g (0,5 Mol) 1,2-Epoxydodecan (Epoxidzahl 8,51, Molgewicht 188,0) in einer Portion zu und rührte 6 Stunden lang. Bereits nach 3 Stunden war eine homogene, klare Lösung (Epoxidzahl = 0) entstanden. Die Analyse der Lösung ergab pro 100 g Lösung 94,8 mval 2-Hydroxydodecyl-2-hydroxyethyldimethylammonium-dihydrogenborat.

### Beispiele 2 bis 8

In gleicher Weise wie zuvor beschrieben wurden das Phenolat, das tertiäre Phosphat, das -dinatriumphosphat, das -natriumhydrogenphosphat, das -natriumcarbonat und das -natriumsulfit sowie das N,N'-Bis-(2-hydroxydodecyl)-tetramethylpropylendiammonium-bis-(natriumhydrogenphosphat) hergestellt (Einzelheiten siehe Tabelle 1).

...

Beispiel 9

In analoger Weise wurde aus 92,7 g (0,5 Mol) Dimethyldecylamin, 80,6 g (0,5 Mol) 1,2-Epoxydecan (Epoxidzahl 9,93, Molgewicht 161,1) und 30,9 g (0,5 Mol) Borsäure in 602,8 g Wasser Decyl-2-hydroxydecyl-dimethylammoniumdihydrogenborat in 25%iger Lösung hergestellt (Einzelheiten siehe Tabelle 1). Die erfindungsgemäß hergestellte Verbindung hat gute antimikrobielle Wirkung, insbesondere gegen Pseudomonas Aeroginosa.

...

TABELLE 1

| Beispiel | Verhältnis Epoxid/Amin/Säure | saure Verbindung | Klare Lösung nach Stunden | Beschaffenheit der Lösung | Besonderheiten |
|---|---|---|---|---|---|
| 2 | 1 : 1 : 1 | Phenol | 0,25 | gel-artig | |
| 3 | 3 : 3 : 1 | $H_3PO_4$ | 2 | klar | |
| 4 | 1 : 1 : 1 | $Na_2HPO_4$ | 0,5 | klar, gelblich | |
| 5 | 1 : 1 : 1 | $NaH_2PO_4$ | 4 | klar, gelblich | |
| 6 | 1 : 1 : 1 | $NaHCO_3$ | 0,75 | klar, gelb | |
| 7 | 1 : 1 : 1 | $NaHSO_3$ | 2 | klar | nach Abkühlen: milchig |
| 8 | 2 : 1 : 2 | $NaH_2PO_4$ | 4 | klar, gelb-braun | Amin-Komponente: Tetramethyl-propylendiamin |
| 9 | 1 : 1 : 1 | $H_3BO_3$ | 2 | klar | Amin-Komponente: Dimethyldecyl-amin; Epoxid-Komponente: 1,2-Epoxydecan |

## PATENTANSPRÜCHE

1. Verfahren zur Herstellung von quartären Ammoniumverbindungen, die wenigstens einen langkettigen Hydroxyalkylrest enthalten, durch Umsetzen eines Salzes eines tertiären Amins mit einer den langkettigen Hydroxyalkylrest einführenden Epoxidverbindung in wäßriger Phase bei erhöhter Temperatur, dadurch gekennzeichnet, daß man das Salz eines tertiären Amins und einer anorganischen oder organischen Säure in Wasser mit einer Epoxidverbindung, die wenigstens eine endständige Epoxygruppe und wenigstens 6 Kohlenstoffatome aufweist, in stöchiometrischem Verhältnis bei Normaldruck und einer Temperatur zwischen 40 und 100 °C umsetzt, wobei die wäßrige Phase des zweiphasigen Reaktionsgemisches vor Beginn der Umsetzung einen pH- Wert von wenigstens 7 aufweist.

2. Verfahren nach Anspruch 1, dadurch gekennzeichnet, daß das tertiäre Amin 1 bis 3 tertiäre Stickstoffatome, insbesondere 1 oder 2 tertiäre Stickstoffatome aufweist.

3. Verfahren nach den Ansprüchen 1 und 2, dadurch gekennzeichnet, daß das tertiäre Amin Alkyl-, Hydroxyalkyl-, Alkenyl- oder Alkylen-Gruppen mit jeweils höchstens 4, insbesondere höchstens 3 Kohlenstoffatomen enthält.

4. Verfahren nach den Ansprüchen 1 bis 3, dadurch gekennzeichnet, daß das tertiäre Amin Dimethylethanolamin oder Tetramethylpropylendiamin ist.

...

5. Verfahren nach den Ansprüchen 1 bis 4, dadurch gekennzeichnet, daß die Säure ausgewählt aus der Gruppe Borsäure, Kohlensäure, Phosphorsäure, Phenol, Caprinsäure und sauren Salzen der Kohlensäure, Phosphorsäure und schwefeligen Säure ist.

6. Verfahren nach den Ansprüchen 1 bis 5, dadurch gekennzeichnet, daß man als Epoxidverbindung entweder ein Epoxyalkan der allgemeinen Formel I

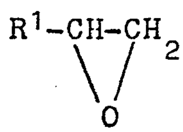 (I)

in der $R^1$ eine lineare oder verzweigte aliphatische Kohlenwasserstoffgruppe mit 4 bis 24 Kohlenstoffatomen oder eine Gruppe der allgemeinen Formel II

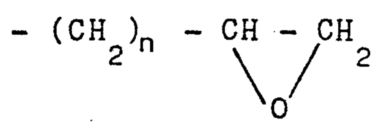

mit n = 4 bis 16 bedeutet, oder einen Glycidether der allgemeinen Formel

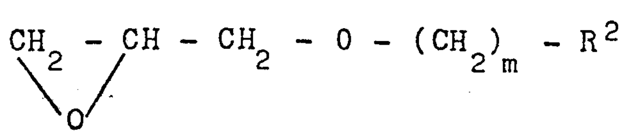

in der m einen Wert von 1 bis 10 und $R^2$ Wasserstoff

...

oder eine aliphatische Kohlenwasserstoffgruppe mit 1 bis 24 Kohlenstoffatomen oder eine Gruppe der allgemeinen Formel IV

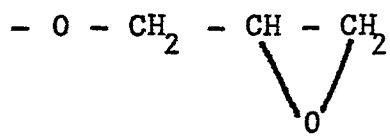 (IV)

bedeutet, mit der Maßgabe, daß der Glycidether der Formel III wenigstens 6 Kohlenstoffatomen enthält, mit dem Aminsalz umsetzt.

7. Verfahren nach den Ansprüchen 1 bis 6, dadurch gekennzeichnet, daß man eine Epoxidverbindung der Formel I, in der $R^1$ eine aliphatische Kohlenwasserstoffgruppe mit 8 bis 18 Kohlenstoffatomen bedeutet, mit einem tertiären Amin, das eine lineare Alkyl- oder Alkenylgruppe mit 10 bis 20 Kohlenstoffatomen und im übrigen zwei $C_1$ - bis $C_4$ -Alkylgruppen aufweist, umsetzt.

8. Verwendung der nach dem Verfahren der Ansprüche 1 bis 6 hergestellten Verbindungen als Antimikrobika bzw. als Textilweichmacher oder -antistatika.

9. Verwendung der nach dem Verfahren von Anspruch 7 hergestellten Verbindungen als Textilweichmacher.